# EUROPEAN PATENT SPECIFICATION

(11) **EP 2 167 958 B1**
(45) Date of publication and mention of the grant of the patent: **01.02.2012**
(21) Application number: 08794243.9
(22) Date of filing: 14.07.2008
(51) Int. Cl.: G01N 33/50

(54) **SOLUTION FOR DETERMINING OVULATORY PERIOD IN WOMEN FROM A TEST TUBE OF URINE**
LÖSUNG ZUR BESTIMMUNG DES FOLLIKELSPRUNGZEITRAUMS BEI FRAUEN MITHILFE EINES URINPROBERÖHRCHENS
SOLUTION POUR DETERMINER UNE PERIODE D'OVULATION CHEZ LES FEMMES A PARTIR D'UN TUBE A ESSAI D'URINE

(30) Priority: 17.07.2007 SK 982007
(43) Date of publication of application: 31.03.2010
(73) Proprietor: Beller, Imrich, 955 01 Topol'cany (SK); Beller, Robert, 949 01 Nitra (SK)
(72) Inventor: Beller, Imrich, 955 01 Topol'cany (SK); Beller, Robert, 949 01 Nitra (SK)
(74) Representative: Neuschl, Jozef
(86) International application number: PCT/SK2008/000009
(87) International publication number: WO 2009/011664

(56) References cited:
- CHEMICAL ABSTRACTS, vol. 131, no. 14, 4 October 1999 (1999-10-04), Columbus, Ohio, US; abstract no.: 181960, IMRICH BELLER: "Two-component agent for determining the optimal fertilization time using urine in women." XP002495166 & SK 279 304 B6 (IMRICH BELLER) 9 September 1998 (1998-09-09) cited in the application
- MOGHISSI K S: "Ovulation detection" ENDOCRINOLOGY AND METABOLISM CLINICS OF NORTH AMERICA, W.B. SAUNDERS COMPANY, PHILADELPHIA, vol. 21, no. 1, 1 March 1992 (1992-03-01), pages 39-55, XP008096229 ISSN: 0889-8529

## Description

### Technical Field

The invention refers to a solution, which causes a direct bond of free SO₄²⁻ anions in a sample of urine to the anion, corresponding cation, acid and carrier (proteins), added by the solution covered by the invention, in an acidic environment.

### Background Art

The solution covered by the invention can be characterised in brief as an indirect contraceptive. In comparison to any other existing contraceptive methods and forms, of which approximately 95% achieves their contraceptive effect by intervening with the female organism, resulting in certain direct effects on female health or even on their children (mainly in synthetic hormonal preparations, intrauterine body), the solution covered by the invention shows properties of an ideal contraceptive in terms of effectiveness.

A Combined Aid to Determine the Optimum Time for Fertilization in Women from Urine - Patent SK No. 279 304:
This aid belongs to natural contraceptive methods because ovulation is determined using a test tube with a sample of urine outside the female body. The determination by analysis is very simple and sufficiently fast - it takes only 15minutes or 30 in maximum.

The first component of the aid contains from 1 to 3.0 % by mass of hydrate salt of sulphate ion SO₄²⁻ with chemical element of bivalent cation of copper or zinc, manganese, cobalt, magnesium, from 1.5 to 2.5 % by volume of acetic acid or butyric acid, oxalic acid, palmitic acid, formic acid, sulphuric acid, hydrochloric acid, and from 94.5 to 97.5 % by volume of water, with overall pH of the solution component from 1.5 to 2.7.

The second component of the aid contains from 1.0 to 4.0 by mass of casein protein and from 96.0 to 99.0 % by volume of water, with overall pH of the solution component from 2.0 to 2.6.

With regard to this, it has to be said that the ovulatory period is a stable physiological process that creates identical biochemical processes and their respective metabolites in every healthy female organism.

Compositions of the solution (covered by the invention) are therefore given by these processes and create identical or similar manifestations just like ovulatory period indicators or ovulation indicators do.

Further development and improvement of this method as a contraceptive required development of a single-ingredient solution compared to a combined solution.

The carrier - casein in the combined solution was not able to bind with the anion (sulphate - SO₄) because a chemical reaction - precipitation occurred immediately upon contact. To produce a single-ingredient solution it was necessary to find an appropriate - optimum anion (with corresponding cations and acids) that would permanently sustain the mutual bond with the carrier.

### Summary of Invention

The disadvantages of existing specific methods of contraception and the current state of technology eliminate the use of the solution covered by the invention to determine the entire ovulatory period in women from a test tube of urine (without any intervention to the female organism), the essence of which is that the solution contains from 5 g to 90 g of hydrate salt of chloride anion Cl⁻ with a chemical element of a bivalent cation of cobalt or copper, magnesium, manganese, zinc, calcium or a monovalent cation of sodium, potassium; from 5 ml to 105 ml of oxalic acid or formic acid, acetic acid, butyric acid, citric acid, lactic acid, hydrofluoric acid, boric acid, tartaric acid; from 2 g to 80 g of casein and the rest up to 1000 ml is water. The overall pH of the solution is 0.8 to 3.5.

The solution includes a single ingredient (more components). It can be used by any woman having a healthy sexual reproduction cycle; samples are easy to take for analysis and the analytical procedure is simple without any apparatus or equipment. The analysis can be done immediately after taking a sample without any treatments or requirements for special rooms and qualifications. A single piece of information by reading or word is sufficient. Results of the analysis are unbiased without any subjective doubts and they are known in 5 minutes, maximum in 30 minutes. The test is qualitative.

The effect of the solution is manifested by a direct bond of free sulphate SO₄²⁻ anions in a sample of urine to the Cl⁻ anion, cation, acid and the carrier, added by the solution covered by the invention, in an acidic environment. Effects of components of the solution will form a precipitate in this reaction.

Presence of free sulphate SO₄²⁻ anions in urine during the test can be seen in dispersion of the initial precipitate in the liquid along the whole test tube and this precipitate starts to float with increasing amounts of free sulphate anions in urine. If the sulphate SO₄²⁻ anions are bound with cations in the urine, the precipitate descends to the bottom of the test tube.

The greatest benefit of the invention is identification of the optimum anion - chloride Cl⁻ that is combined with the carrier (casein) at the time of manufacture (reconstitution) of the solution, but also permanently with corresponding cations and acids. This ensures that all the ingredients of the solution are added to a single complete set - i.e. they create a single-ingredient solution used to determine ovulatory period in women from a test tube of urine. This increases its reliability and allows concurrent effects of every solution ingredient during analysis. This improves qualitative level of results of the analysis and avoids errors in the order of solutions during analysis.

In a healthy female organism, the bond between some cations and anions of sulphate SO₄²⁻ is broken at the time of ovulation.

This condition is supported by biochemical changes in the body and affects successful fertilization, and this where effects of free SO₄²⁻ anions and some cations on these processes and subsequent pregnancy can be seen especially in the sulphate-cation bond, thus in transfer of ions into cells by means of aldosterone, a hormone released in higher amounts during ovulation and during the entire period of pregnancy.

This specifies and extends another significant function of these bivalent cations in the field of reproduction. So far, they have been defined only in terms of health and they have been said to be important and needed for reproduction.

Investigation of the optimum combination - bonds of the solution covered by the invention resulted in development of a new method of contraception that has become permanently established for optimum determination of ovulatory period in women, as BIOCHEMICAL METHOD OF CONTRACEPTION

This is possible due to its properties - high effectiveness and reliability and mainly due to the fact that this is the only method (at present) that satisfies all the requirements of optimum contraception (in global terms) for every group of women - in terms of health, social status, religion, age etc.

The method is optimal because it uses and determines biochemical indicators (metabolites) resulting from physiological changes in the female body at the ovulatory period, i.e. the metabolites become the basic active substance - material for the significant working method.

Biochemical metabolites of the ovulatory load in the female body are the most optimum products for determining the ovulatory period. Compared to any other existing method for determining ovulation (at present), they are the most reliable and allow to determine the entire ovulatory period by the most objective and most distinct manifestation of assessment test, i.e. objective manifestation of phases: initial - main ovulatory - final.

### Examples

The invention will be designed specifically as a solution, where -1000 ml of the solution contains:
- 20 g of hydrate salt of cobalt (II) chloride
   80 ml of oxalic acid
   20 g f casein
   880 ml of water
   pH of the solution is 0.8
- 35 g of hydrate salt of calcium chloride
   24 ml of acetic acid
   80 g of casein
   861 ml of water
   pH of the solution is 2.4
- 90 g of hydrate salt of magnesium chloride
   31 ml of butyric acid
   72 g of casein
   807 ml of water
   pH of the solution is 3.5
- 5 g of hydrate salt of manganese chloride
   18 ml of formic acid
   63 g of casein
   914 ml of water
   pH of the solution is 1.9
- 72 g of hydrate salt of sodium chloride
   44 ml of lactic acid
   14 g of casein
   870 ml of water
   pH of the solution is 2.7
- 25 g of hydrate salt of zinc chloride
   92 ml of tartaric acid
   51 g of casein
   832 ml of water
   pH of the solution is 3.0
- 18 g of hydrate salt of calcium chloride
   72 ml of citric acid
   46 g of casein
   864 ml of water
   pH of the solution is 2.6
- 84 g of hydrate salt of copper chloride
   67 ml of boric acid
   32 g of casein
   817 ml of water
   pH of the solution is 3.1
- 50 g of hydrate salt of sodium chloride
   105 ml of hydrofluoric acid
   2.0 g of casein
   843 ml of water
   pH of the solution is 2.8
- 46 g of hydrate salt of potassium chloride
   57 ml of formic acid
   27 g of casein
   870 ml of water
   pH of the solution is 2.4
- 62 g of hydrate salt of magnesium chloride
   5.0 ml of lactic acid
   58 g of casein
   875 ml of water
   pH of the solution is 2.9

The examples above are just one of the numerous possible combinations of the total number of possible combinations of hydrate salt of chloride Cl⁻ anion with the mentioned chemical components in bivalent or monovalent cations and specific acids in terms of their percentage range.

### Feasibility of Test with the Solution Covered by the Invention

Put 2.0 ml of urine into a test tube, add 1.0 ml of the solution covered by the invention and mix 2-3 times by reverting the test tube by 180 °. Put the test tube to the vertical position to avoid it from turning and to allow dispersion of the precipitate to a relevant position for 5 minutes or 30 minutes in maximum to determine the result of the test.

The result is evaluated according to the position of the precipitate formed in the test tube.
- The precipitate descends to the bottom of the test tube - no fertilisation;
- The precipitation does not descend and does not float in the liquid - it is dispersed along the liquid - appropriate time for fertilisation begins (a clear liquid is usually seen at the bottom of the solution), it lasts 1 day or ends (a clear liquid is usually seen at the top of the solution) - lasts 1 day, too. However, fertilisation might happen in both cases - due to egg and sperm lifecycles;
- The precipitate floats at the top of the liquid in the test tube - time appropriate for fertilisation - lasts 2 days.

During analysis, the solution works on the qualitative level, so accuracy of analysis is simpler (minor deviations in dosing have no impact on results) than in quantitative analyses.

Special monitoring of woman's own ovulatory cycles (not menstrual cycles) in individual months makes it possible for every woman to prevent undesired pregnancy. The product allows every woman to become familiar and monitor her own physiological process of ovulatory period and its validity as needed.

### Industrial Applicability

Based on the invention, the solution should be used primarily to determine course of ovulatory cycles in individual months in women, with major focus on determining their ovulatory period as an appropriate time for fertilisation, but mostly as a protection against conception. This allows a personal and very simple monitoring of qualitative physiological course of the ovulatory period according to personal needs and to choose necessary treatment in case of any deviations.

It can be used also for detection of early pregnancy for information purposes by checking existence of the following ovulatory period.

The possibility of determining a valid ovulatory period should be verified carefully especially in adolescent girls as a prevention, where possible and early treatment can help prevent serious disorders that are difficult to treat in adult age, or that can be treated only partially.

The solution covered by the invention allows a successful monitoring of onset and course of menopause, where only the first stage of the ovulatory period is manifested - the analysed precipitate is dispersed only, but it does not float, i.e. there is no ovulation - no egg is produced.

## Claims

1. The solution to determine the ovulatory period in women from a test tube of urine, **characterised by** the fact that 1000 ml of the solution contains from 5 to 90 g of hydrate salt of chloride anion Cl⁻ with a chemical element of bivalent cation of cobalt or copper, magnesium, manganese, zinc, calcium or a monovalent cation of sodium, potassium; from 5 to 105 ml of oxalic acid or formic acid, acetic acid, butyric acid, citric acid, lactic acid, hydrofluoric acid, boric acid, tartaric acid; from 2 to 80 g of casein and the rest up to 1000 ml is water, with overall pH of the solution 0.8 to 3.5.

## Patentansprüche

1. Die Lösung zur Bestimmung der Ovulationsperiode bei Frauen aus einem Reagenzglas vom Harn, **dadurch gekennzeichnet, dass** 1000 ml der Lösung 5 bis 90 g eines Hydratsalzes des Chlorid-Anions Cl⁻ mit einem chemischen Element des bivalenten Kation von Kobalt oder Kupfer, Magnesium, Mangan, Zink, Calcium, oder monovalenten Kation von Natrium, Kalium; 5 bis 105 ml Oxalsäure oder Ameisensäure, Essigsäure, Buttersäure, Zitronensäure, Milchsäure, Hydrofluorsäure, Borsäure, Weinsäure; 2 bis 80 g Kaseins enthält und der Rest bis 1000 ml Wasser ist, mit gesamten pH der Lösung 0,8 bis 3,5.

## Revendications

1. La solution pour déterminer la période d'ovulation chez la femme d'une tube à essai de l'urine, **caractérisée en ce que** 1000 ml de la solution contient de 5 à 90 g d'un sel hydrate de l'anion chlorure Cl⁻ avec un élélment chimique d'un cation bivalent de cobalt ou cuivre, magnésium, manganèse, zinc, calcium, ou un cation monovalent de natrium, potaasium ; de 5 à 105 ml de l'acide oxalique ou l'acide formique, l'acide acétique, l'acide butyrique, l'acide citrique, l'acide lactique, l'acide hydrofluorique, l'acide borique, l'acide tartrique ; de 2 à 80 g de la caséine, le résidu jusqu'à 1000 ml étant l'eau, avec pH global de la solution de 0,8 à 3,5.
